# EUROPÄISCHE PATENTSCHRIFT

(11) **EP 1 480 586 B1**
(45) Veröffentlichungstag und Bekanntmachung des Hinweises auf die Patenterteilung: **21.02.2007**
(21) Anmeldenummer: 03720165.4
(22) Anmeldetag: 06.03.2003
(51) Int. Cl.: A61F 5/41

(54) **VORRICHTUNG ZUR DAUERHAFTEN VERLÄNGERUNG LANGGESTRECKTER KÖRPERTEILE**
DEVICE FOR PERMANENTLY EXTENDING ELONGATE BODY PARTS
DISPOSITIF POUR ALLONGER DE MANIERE DURABLE DES PARTIES CORPORELLES OBLONGUES

(30) Priorität: 07.03.2002 DE 20203927 U
(43) Veröffentlichungstag der Anmeldung: 01.12.2004
(73) Patentinhaber: Msp Concept Gmbh & Co. Kg, 10781 Berlin (DE)
(72) Erfinder: SUCHY, Matthias, 10719 Berlin (DE); SUCHY, Sylvia, 10719 Berlin (DE)
(74) Vertreter: Specht, Volker
(86) Internationale Anmeldenummer: PCT/DE2003/000812
(87) Internationale Veröffentlichungsnummer: WO 2003/073967

(56) Entgegenhaltungen:
- WO-A-96/26691
- WO-A-97/28764
- DE-A- 19 618 352
- DE-U- 29 521 655
- US-A- 5 642 557

## Beschreibung

Die Erfindung betrifft eine Vorrichtung zur dauerhaften Verlängerung langgestreckter Körperteile, insbesondere des Penis, die einen Stützring, mindestens eine in dem Stützring gelenkig befestigte, axial federnd gelagerte und graduell längenverstellbare Streckstange sowie ein am distalen Ende der Streckstange angebrachtes Befestigungsmittel umfasst. Eine Vorrichtung gemäß Oberbegriff von Anspruch 1 ist z.B. aus WO-A-96/26691 bekannt. Derartige Streckgeräte, mit deren Hilfe es ohne operativen Eingriff möglich ist, auf der Grundlage einer dauernden Dehnung des Gewebes durch Einwirkung einer Streckkraft eine bleibende Verlängerung bestimmter Körperteile, zum Beispiel des Penis, zu erzielen, sind bekannt. Beispielsweise wird in der DE 196 18 351 A1 eine Vorrichtung zur Penisverlängerung beschrieben, bei der die Streckelemente teleskopisch ineinander geführte Streckstangen sind, die in der jeweils gewünschten Länge arretierbar sind. Als Befestigungsmittel am distalen Streckstangenende ist eine Aufnahmeschale mit einer an dieser in variablem Umfang einstellbaren Halteschlaufe vorgesehen. Diese Vorrichtung ist insofern nachteilig, als eine allmähliche feindosierte Erhöhung der Streckkraft nicht möglich ist und die schlaufenartige Halterung des Penis mit Verletzungsgefahren bei der Benutzung des Gerätes verbunden ist.

Bei einer anderen Ausführungsform eines Penisextensionsgerätes gemäß der DE 295 21 655 U1 ist die jeweilige Streckstange aus zwei Gewindehülsen mit Innengewinde, die durch eine Gewindestange verbunden sind, gebildet. Dadurch ist eine Feineinstellung der Streckkraft bzw. der Länge der Streckstangen möglich. Die Streckstangen sind zudem schwenkbar und federnd in dem Stützring gelagert. Als Befestigungsmittel dient ein den Penis umschlingendes breites Band mit zwei im Abstand der Streckstangen ausgebildeten zylindrischen Taschen, in die die Streckstangen eingefügt werden. Abgesehen von der Verletzungsgefahr durch die Bandbefestigung bereitet hier auch das Anlegen des Gerätes Schwierigkeiten bzw. bedarf einer erheblichen Geschicklichkeit.

Bei einer weiterhin bekannten Streckvorrichtung sind an dem Stützring zwei parallele Gewindestangen gelagert, die jeweils mit einer ein Innengewinde aufweisenden Verstellhülse verschraubt sind. Auf der Verstellhülse ist eine Federhülse mit innen liegender Feder entgegen der Federwirkung teleskopisch verschiebbar gelagert. Die Federhülsen sind durch Verlängerungsstangen verlängerbar. Als Befestigungsmittel für das distale Penisende ist eine Aufnahmeschale mit seitlichen Hülsen zur Steckverbindung mit den freien Enden der Federhülsen bzw. der Verlängerungsstangen sowie einem elastischen Befestigungsband, dessen Enden in veränderlicher Lage in der Aufnahmeschale arretierbar sind, vorgesehen. Auch diese Vorrichtung ist insofern nachteilig, als das Anlegen des Streckgerätes ein hohes Maß an Geschicklichkeit erfordert und in Verbindung mit dem elastischen Befestigungsband zu Verletzungen führen kann.

Der Erfindung liegt die Aufgabe zugrunde, eine Vorrichtung der eingangs erwähnten Art so auszubilden, dass beim Anlegen und bei der ständigen Benutzung eine einfache, komfortable und schmerzfreie Handhabung des Expansionsgerätes gewährleistet ist.

Erfindungsgemäß wird die Aufgabe mit einer gemäß den Merkmalen des Patentanspruchs 1 ausgebildeten Vorrichtung gelöst. Aus den Unteransprüchen ergeben sich weitere Merkmale und vorteilhafte Ausgestaltungen der Erfindung.

Ein wichtiges Erfindungsmerkmal besteht darin, dass das Befestigungsmittel als zylindrisch vorgeformtes, das betreffende Körperteil teilweise oder vollständig elastisch umfassendes Element ausgebildet ist. Die Halterung des Körperteils im Streckzustand erfolgt somit großflächig und weichelastisch und im Wesentlichen am gesamten Umfang. Das gewährleistet aufgrund des großflächigen elastischen Druckes einen hohen Benutzungskomfort. Die Verletzungsgefahr durch Quetschungen wird infolge der annähernd zylindrisch vorgeformten Ausbildung erheblich minimiert.

Am Umfang des Befestigungsmittels sind nach einem weiteren wichtigen Erfindungsmerkmal seitlich an der Streckstange verrastbare, die Streckstangen vorzugsweise an deren Umfang federnd umfassende Halteclips angebracht. Das heißt, es kann zunächst sehr bequem das Befestigungsmittel am Penis angebracht werden, das anschließend mit dem Halteclip nur an den Streckstangen verrastet werden muss. Das Anlegen des Extensionsgerätes wird dadurch wesentlich vereinfacht, ohne dass dabei schmerzhafte Quetschungen oder eine Überstreckung des Körperteils auftreten können. Darüber hinaus kann das Streckgerät zwischenzeitlich auch schnell gelöst und wieder angelegt werden.

Gemäß einem weiteren wichtigen Erfindungsmerkmal sind die Halteclips am in Streckrichtung oberen Rand des Befestigungsmittels angebracht, so dass auch kurze Körperteile erfasst werden können..

In einer Ausführungsvariante umfasst das Befestigungsmittel eine steife Aufnahmeschale mit den daran angeformten Halteclips und ein Spannelement aus einem gewölbten weichelastischen Halteteil mit daran seitlich anschließenden dehnbaren Spannbändern, die in Schlitzen der Aufnahmeschale arretiert werden.

Gemäß einer anderen Ausführungsvariante ist auch die Aufnahmeschale weichelastisch ausgebildet, oder die Aufnahmeschale und/oder das Spannelement sind durch ein Luftkissen elastisch ausgebildet. Vorzugsweise besteht das im Wesentlichen zylindrische Befestigungsmittel aus zwei gelenkig miteinander verbundenen, an der Innenfläche mit einem aufblasbaren Luftpolsterring oder elastischem Material belegten Halbschalen, die mit einem Verschluss in der Weite verstellbar verschlossen sind. Auf diese Weise kann das betreffende Körperteil schnell und schonend mit dem Befestigungsmittel verbunden und in diesem fixiert werden sowie im Bedarfsfall einfach und schnell freigegeben werden.

In einer bevorzugten Variante ist der Luftposterring von Hand oder mit einer Druckluftkartusche aufblasbar. Eine Handpumpe kann in das Befestigungsmittel integriert sein.

In noch weiterer Ausbildung der Erfindung kann das Befestigungsmittel auch als einstückiges zylindrisches, aufblasbares Bauteil mit am Außenumfang angeformten Halteclips ausgeführt sein.

Ein Ausführungsbeispiel der Erfindung wird anhand der Zeichnung näher erläutert. Es zeigen:

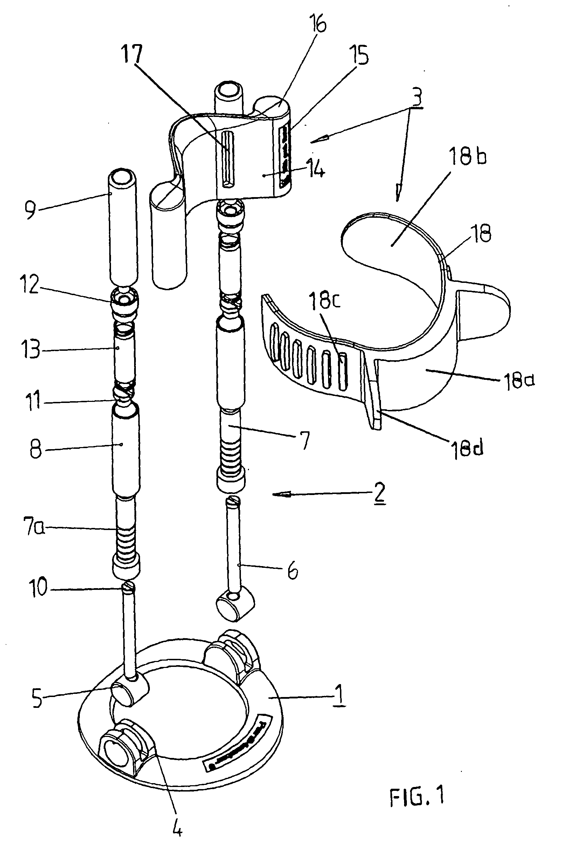

- Fig. 1: eine perspektivische Ansicht einer erfindungsgemäßen Vorrichtung zur dauerhaften Penisverlängerung in einer auseinander gezogenen Darstellung; und

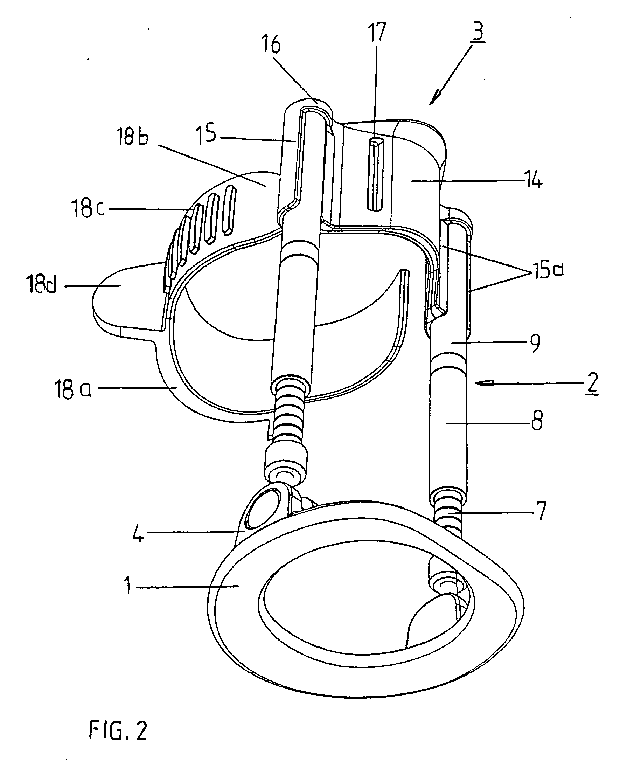

- Fig. 2: eine perspektivische Rückansicht der Vorrichtung nach Fig. 1 in zusammengebautem Zustand.
- Fig. 3: eine perspektivische Ansicht einer Ausführungsform der Aufnahmeschale mit in Streckrichtung nach oben versetzten, sich über die Aufnahmeschale hinaus erstreckenden Halteclips;
- Fig. 4: eine Ansicht eines zylindrischen, aufklappbar ausgebildeten Befestigungsmittels mit luftelastischen Innenflächen und versetzt angeordneten Halteclips; und
- Fig. 5: eine Draufsicht des Befestigungsmittels nach Fig. 4, jedoch mit schaumelastischer Innenauskleidung.

Wie aus der Zeichnung ersichtlich, umfasst die Vorrichtung einen zur Abstützung am Körper des Benutzers vorgesehenen Stützring 1, zwei an dessen Vorderseite parallel angeordnete und schwenkbar gelagerte Streckstangen 2, die graduell verlängerbar und axial federnd ausgebildet sind, sowie ein am distalen Ende der beiden Streckstangen 2 lösbar gehaltenes Befestigungsmittel 3 zum Einspannen und Fixieren des Penis im Bereich der Glans. An der vorderen Fläche des Stützrings 1 sind einander gegenüberliegend zwei Gelenkaufnahmen 4 zur Aufnahme eines in dieser um eine horizontale Achse drehbaren Gelenkstücks 5 angeformt. Die Rückseite des Stützrings 1 bildet eine ebene, geschlossene Fläche, deren Übergang zur gewölbten vorderen Fläche gerundet ist. Die Streckstange 2 besteht aus einer mit dem Gelenkstück 5 verbundenen Gewindestange 6, einer mit dieser über ein Innengewinde verbundenen und mithin axial verstellbaren Verstellhülse 7, einer teleskopisch auf der Verstellhülse 7 angeordneten und zum distalen Ende der Streckstange 2 hin unter Federdruck stehenden Federhülse 8 sowie einer oder mehreren Verlängerungsstangen 9. Auf der Umfangsfläche der Verstellhülse 7 sind Markierungen 7a angebracht, die - entsprechend der Lage der Federhülse 8 auf der Verstellhülse 7 - die Größe der über die Federhülse 8 aufgebrachten Zugspannung anzeigen. Die Gewindestange 6 weist am Ende ein anschraubbares Anschlagstück 10 auf, so dass sich die Verstellhülse 7 beim Verlängern nicht unbeabsichtigt von der Gewindestange 6 lösen kann. In der Federhülse 8, die in der ausgezogenen Lage durch ein stirnseitig mit der Verstellhülse 7 verschraubtes Anschlagstück 11 gehalten ist und durch einen Verschlussadapter 12 verschlossen ist, befindet sich eine Druckfeder 13, die die Federhülse 8 zum distalen Ende der Streckstange 2 hin drückt. In dem Verschlussadapter 12 ist schließlich die Verlängerungsstange 9 verschraubbar. Das im Ausführungsbeispiel zweiteilige Befestigungsmittel 3 besteht aus einer im Bereich der Auflagefläche konkav ausgebildeten, im Wesentlichen biegesteifen Aufnahmeschale 14 mit seitlich angeformten Halteclips 15, die am Ende ein zylindrisches Steckteil 16 aufweisen. Der obere Rand der Aufnahmeschale 14 ist zur Rückseite der Aufnahmeschale hin abgebogen. Mit den in Form eines in Längsrichtung geschlitzten Zylinders ausgeführten Halteclips 15 mit federnden Wangen 15a kann die Aufnahmeschale 14 von der Seite an den Streckstangen 2 angebracht werden, während die Arretierung in Längsrichtung durch das Steckteil 16 erfolgt. In der Aufnahmeschale 14 sind Schlitzöffnungen 17 ausgebildet. Das zweite Teil des Befestigungsmittels 3 besteht aus einem gummielastischen Spannelement 18, das ein entsprechend der Penisform gewölbtes weichelastisches Halteteil 18a mit stark gerundeten Kanten, zwei Spannbänder 18b mit auf deren Oberfläche im Abstand ausgeformten Raststegen 18c zum Arretieren der Spannbänder 18b in den Schlitzöffnungen 17 der Aufnahmeschale 14 und zwei den Spannbändern 18b entgegengerichtete Zuglaschen 18d zum Lösen des Spannelements 18 und zur Begrenzung der Spannkräfte aufweist. Das so ausgebildete Befestigungsmittel kann vom Benutzer unabhängig von den Streckstangen bequem angelegt werden, so dass Verletzungen oder ein schmerzhaftes Einklemmen ausgeschlossen ist. Die schmerzfreie Befestigung wird zudem durch die weichelastische und vorgeformte sowie an den Kanten abgerundete Gestaltung des Halteteils 18a begünstigt. Andererseits verhindern die Zuglaschen 18d das Aufbringen einer zu großen Spannkraft und ermöglichen zudem ein schnelles bequemes Lösen des Spannelements 18. Nach dem Anlegen des Befestigungsmittels 3 werden die Halteclips 15 seitlich an den zuvor angelegten, am Körper über den Stützring 1 abgestützten Streckstangen 2 verrastet. Das zylindrische Steckteil 16 stellt eine zusätzliche Sicherung zur seitlichen Arretierung dar. Es genügt jedoch eine obere Begrenzungsplatte am Halteclip 15, um ein Herunterrutschen des Befestigungsmittels 3 zu vermeiden.

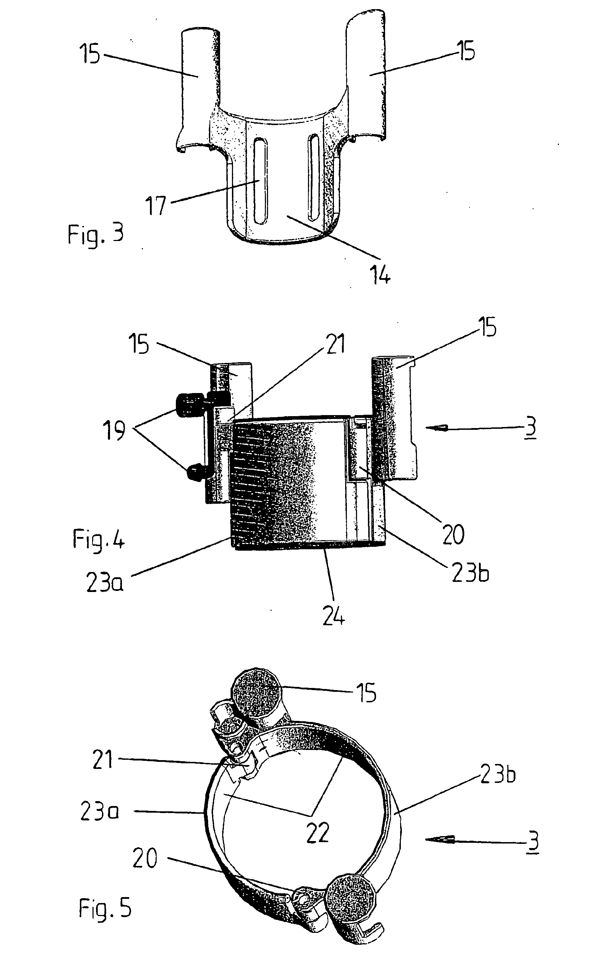

Gemäß einer in Fig. 3 gezeigten bevorzugten Ausführungsform sind die beiden Halteclips 15 am oberen Rand der Aufnahmeschale 14 angeformt. Dadurch kann nach dem Anlegen des Befestigungsmittels 3 auch an einen sehr kurzen Penis die Verbindung zwischen den Halteclips 15 und den Streckstangen ohne Penisüberstreckung hergestellt werden. Die nach oben überstehenden Halteclips 15 dienen gleichzeitig dem Schutz des oberen, freien Penisteils. Die Schlitzöffnungen 17 in der Aufnahmeschale 14 können noch derart langgestreckt ausgebildet sein, dass das Anbringen des elastischen Spannelements 18 in der in Streckrichtung variiert werden kann.

Gemäß einer noch anderen, in der Zeichnung nicht dargestellten Ausführungsvariante kann das Spannelement 18 als band- oder schlauchförmiger, flexibler und aufblasbarer Hohlkörper ausgebildet sein, dessen Enden fest mit der Aufnahmeschale 14 verbunden oder lösbar an dieser gehalten sind. Nach dem Anlegen des so ausgeführten Befestigungsmittels 3 in nicht aufgeblasenem Zustand des aufblasbaren Spannelements wird mit einer externen oder in das Befestigungsmittel integrierten Pumpe oder einer Druckluftkartusche Luft in das aufblasbare Spannelement eingeblasen, um den Penis ohne jegliche Einklemmgefahr dosiert und elastisch schonend zu fixieren. In Ausgestaltung dieser Ausführungsform kann auch die Aufnahmeschale 14 aus einem aufblasbaren Hohlkörper gebildet sein oder das Befestigungsmittel 3 ist insgesamt als doppelwandiger, zylindrischer Hohlkörper mit an der Außenwand diametral gegenüberliegend angebrachten Halteclips 15 ausgeführt. Dabei kann die Außenwand starr und die Innenwand flexibel sein. Es können aber auch beide Wände flexibel sein. Nach dem Aufblasen des zylindrischen doppelwandigen Befestigungsmittels mit mindestens einer flexiblen Innenwand verringert sich der Innendurchmesser und die Innenwand legt sich in dem zu fixierenden Bereich gleichmäßig und elastisch am gesamten Umfang des Penis an. In einer noch weiteren, in der Zeichnung ebenfalls nicht dargestellten Variante kann am distalen Rand des jeweiligen Befestigungsmittels eine ganz oder teilweise umlaufende elastische Wulst angeformt sein, um die Fixierung in axialer Richtung weiter zu optimieren.

Eine bevorzugte Ausführungsvariante eines im Wesentlichen kreisförmigen, am gesamten Umfang des Penis elastisch anliegenden, zylindrisch ausgebildeten Befestigungsmittels 3 mit in Streckrichtung versetzten Halteclips 15 ist in den Figuren 4 und 5 wiedergegeben. Das Befestigungsmittel 3 besteht in diesem Fall aus zwei über ein Scharnier 20 und einen Verschluss 21 verbundenen starren Halbschalen 23a, 23b. In der Ausführungsform nach Fig. 4 ist an den Innenflächen der Halbschalen 23a, 23b ein aufblasbarer, im Bereich des Verschlusses 21 geteilter Luftpolsterring 24 mit an der Außenseite vorgesehenen Ventilen 19 zum Zuführen und Ablassen von Luft befestigt ist. Gemäß der in Fig. 5 gezeigten Variante ist an der Innenfläche der beiden starren Halbschalen 23a, 23b ein elastisches Gel oder eine Schaumstoffschicht 22 angebracht, die im Bereich des Verschlusses 21 unterbrochen ist, so dass die obere Halbschale 23a vollständig von der unteren Halbschale 23b weggeschwenkt werden kann. Die Halbschalen 23a, 23b sind unterschiedlich groß, wobei an der größeren Halbschale 23b die beiden Halbschalen 15 in Streckrichtung nach oben versetzt angebracht sind. Der Verschluss 21 kann derart verstellbar ausgebildet sein, dass der Innenumfang des Befestigungsmittels an den Penisumfang angepasst werden kann, beispielsweise durch ein Rast-, Druckknopf- oder Klettbandverschlusssystem, und eine sichere und schonende Fixierung erreicht wird. Das Aufblasen des Luftposterrings 24 kann mit einer integrierten oder externen, elektrisch oder manuell betrieben Luftpumpe, mit einer Druckluftkartusche oder auch mit Atemluft erfolgen.

### Bezugszeichenliste

- a.: Stützring
- b.: Streckstange
- c.: Befestigungsmittel
- d.: Gelenkaufnahme
- e.: Gelenkstück
- f.: Gewindestange
- g.: Verstellhülse
- 7a: Markierung
- h.: Federhülse
- i.: Verlängerungsstange
- j.: Anschlagstück
- k.: Anschlagstück
- l.: Verschlussadapter
- m.: Druckfeder
- n.: Aufnahmeschale
- o.: Halteclip
- 15a: federnde Wangen
- p.: Anschlagplatte/zyl. Steckteil
- q.: Schlitzöffnungen
- r.: Elastisches Spannelement
- 18a: weichelast. gewölbtes Halteteil
- 18b: Spannbänder
- 18c: Raststege
- 18d: Zuglaschen
- s.: Ein-/Auslassventil
- t.: Scharnier
- u.: Verschluss
- v.: elastischer Schaumstoff/Gel
- 23a: kleine Halbschale
- 23b: große Halbschale
- 24: Luftposterring

## Patentansprüche

1. Vorrichtung zur dauerhaften Verlängerung langgestreckter Körperteile, insbesondere des Penis, die einen Stützring (1), mindestens eine am Stützring (1) gelenkig befestigte, axial federnd gelagerte und graduell längenverstellbare Streckstange (2) sowie ein am distalen Ende der Streckstange (2) gehaltenes, das Körperteil umspannendes Befestigungsmittel (3) umfasst, **dadurch gekennzeichnet, dass** das Befestigungsmittel (3) als im Wesentlichen zylindrisch vorgeformtes und das betreffende Körperteil ganz oder teilweise weichelastisch umschließendes Bauteil (14, 18; 22 bis 24) mit nach dem Anlegen des Befestigungsmittels (3) seitlich am Umfang der Streckstange (2) verrastbaren Halteclips (15) ausgebildet ist.

2. Vorrichtung nach Anspruch 1, **dadurch gekennzeichnet, dass** der mindestens eine Halteclip (15) als in Längsrichtung durchgehend geschlitzter Zylinder mit federnden Seitenwangen (15a) und einer distalen Anschlagplatte (16) ausgebildet ist.

3. Vorrichtung nach Anspruch 1, **dadurch gekennzeichnet, dass** die Halteclips (15) vom in Streckrichtung distalen Bereich des Befestigungsmittels (3) ausgehen und sich über dessen distales Ende hinaus erstrecken.

4. Vorrichtung nach Anspruch 1, **dadurch gekennzeichnet, dass** das Befestigungsmittel (3) aus einer konkav gewölbten Aufnahmeschale (14) mit den seitlich vom distalen Ende ausgehenden Halteclips (15) und einem elastischen Spannelement (18) besteht.

5. Vorrichtung nach Anspruch 4, **dadurch gekennzeichnet, dass** das Spannelement (18) aus einem gewölbt vorgeformten weichelastischen Halteteil (18a) mit von dessen Enden ausgehenden elastisch dehnbaren Spannbändern (18b) besteht, wobei an den Außenflächen der Spannbänder (18b) Raststege (18c) zum Arretieren der Spannbänder in Schlitzöffnungen (17) der Aufnahmeschale (14) und Zuglaschen (18d) zum Lösen der Spannbänder (18b) und zum Begrenzen der Spannkräfte angeformt sind.

6. Vorrichtung nach Anspruch 5, **dadurch gekennzeichnet, dass** die Raststege (18c) und die Schlitzöffnungen (17) abgerundete Kanten haben.

7. Vorrichtung nach Anspruch 5, **dadurch gekennzeichnet, dass** die Stärke des gewölbten Halteteils (18a) um ein Mehrfaches größer als die der elastischen Spannbänder (18b) ist.

8. Vorrichtung nach Anspruch 5, **dadurch gekennzeichnet, dass** das Spannelement (18) in Längsrichtung variabel an der Aufnahmeschale (14) fixierbar ist, wobei die Länge der Schlitzöffnungen (17) größer als die Breite des Spannbandes (18b) ist.

9. Vorrichtung nach Anspruch 1, **dadurch gekennzeichnet, dass** das zylindrische Befestigungsmittel (3) aus zwei über ein Scharnier (20) und einen Verschluss (21) verbundenen, einen Zylinder bildenden Halbschalen (23a, 23b) besteht, an deren Innenflächen ein hochelastisches Material (22, 24) angebracht ist.

10. Vorrichtung nach Anspruch 9, **dadurch gekennzeichnet, dass** das hochelastische Material ein aufblasbarer, im Bereich des Verschlusses (21) geteilter Luftpolsterring (24) ist.

11. Vorrichtung nach einem der Anspruch 10, **dadurch gekennzeichnet, dass** in der Wand des Luftpolsterrings (24) ein Ein- und Auslassventil (19) angeordnet ist und zum Aufblasen des aufblasbaren Teils eine externe Pumpe oder Druckluftkartusche oder eine in das Befestigungsmittel (3) integrierte, manuell betätigbare Pumpe oder Druckluftkartusche vorgesehen ist.

12. Vorrichtung nach Anspruch 9, **dadurch gekennzeichnet, dass** das hochelastische Material ein Schaumstoff oder Gel (22) ist.

13. Vorrichtung nach Anspruch 9, **dadurch gekennzeichnet, dass** die beiden Halbschalen (23a, 23b) unterschiedlich groß sind, wobei die Halteclips (15) an der größeren Halbschale (23b) angebracht sind.

14. Vorrichtung nach Anspruch 9, **dadurch gekennzeichnet, dass** der Verschluss (21) zur Einstellung der Größe des von den beiden Halbschalen (23a, 23b) gebildeten Innendurchmessers verstellbar ausgebildet ist.

15. Vorrichtung nach Anspruch 14, **dadurch gekennzeichnet, dass** der verstellbare Verschluss (21) durch ein Rast-, Druckknopf- oder KlettbandVerschlusssystem gebildet ist.

16. Vorrichtung nach Anspruch 1, **dadurch gekennzeichnet, dass** das Befestigungsmittel (3) als einstückiges zylindrisches, doppelwandiges, aufblasbares Bauteil mit flexibler Innenwand und flexibler oder starrer Außenwand und an der Außenwand angebrachtem Halteclip (15) ausgebildet ist, das ein Einlass- und ein Auslassventil (19) zum Einblasen und Ablassen von Luft aufweist.

17. Vorrichtung nach Anspruch 1, **dadurch gekennzeichnet, dass** die Streckstangen (2) über ein Kugelgelenk am Stützring (1) gehalten sind und die Halteclips (15) gelenkig am Befestigungsmittel (3) angebracht sind.

18. Vorrichtung nach Anspruch 1, **dadurch gekennzeichnet, dass** die Streckstange (2) zur elastischen Längenänderung aus einer Gewindestange (6), einer mit dieser verschraubten Verstellhülse (7) und einer diese teleskopisch umfassenden, federnd gelagerten Federhülse (8) besteht, wobei am distalen Ende der Gewindestange (6) ein Anschlagstück (10) zur Vermeidung des vollständigen Herausdrehens der Verstellhülse (7) ausgebildet ist.

19. Vorrichtung nach Anspruch 18, **dadurch gekennzeichnet, dass** am Umfang der Verstellhülse (7) zur Anzeige der von der Federhülse (8) erzeugten Spannkraft Markierungen (7a) vorgesehen sind.

20. Vorrichtung nach Anspruch 18, **dadurch gekennzeichnet, dass** die Streckstange (2) aus einer Mehrzahl miteinander verschraubbarer Verlängerungsstangen (9) in unterschiedlicher Länge zusammensetzbar ist.

## Claims

1. A device for permanently extending elongate body parts, particularly the penis, comprising a support ring (1), at least one stretching rod (2) coupled to the support ring and spring-mounted in axial direction that can gradually be adjusted in length, and fixing means (3) retained on the distal end of the stretching rod and embracing the the body part, **characterized in that** the fixing means (3) as a substantially cylindrical preformed component (14, 18; 22 to 24) that fully or partially and soft-flexibly surrounds the respective body part is provided with retaining clips (15) locking sideways into the stretch rod (2) after putting on the fixing means (3).

2. The device according to claim 1, **characterized in that** said at least one retaining clip (15) is designed as a continuously slotted cylinder with flexible cheeks (15a) and a distal stop plate (16).

3. The device according to claim 1, **characterized in that** the retaining clips (15) extend from the distal section of the fixing means (3) in stretching direction and beyond its distal end.

4. The device according to claim 1, **characterized in that** the fixing means (3) consists of a concave receiving shell (14) with retaining clips (15) extending from its sides at the distal end and an elastic fastening element (18).

5. The device according to claim 4, **characterized in that** the fastening element (18) consists of a domed preformed flexible support part (18a) from the ends of which extend elastic fastening straps (18b), the outer surfaces of said fastening straps (18b) comprising latches (18c) for locking the fastening straps into slots (17) of the receiving shell (14) and shackles (18d) for releasing the fastening straps (18b) and for limiting tension forces.

6. The device according to claim 5, **characterized in that** the latches (18c) and slots (17) have rounded edges.

7. The device according to claim 5, **characterized in that** the thickness of the domed support part (18a) is multiple times greater than that of the elastic fastening straps (18b).

8. The device according to claim 5, **characterized in that** the fastening element (18) can be adjusted in longitudinal direction by variably fixing it to the receiving shell (14), the length of the slots (17) exceeding the width of the fastening strap (18b).

9. The device according to claim 1, **characterized in that** the cylindrical fixing means (3) consists of two shells (23a, 23b) connected by a hinge (20) and a lock (21) and forming a cylinder, and **in that** a highly elastic material (22, 24) is applied to the inner surfaces of said shells.

10. The device according to claim 9, **characterized in that** said highly elastic material is an inflatable air cushion ring (24) that is split in the section of the lock (21).

11. The device according to claim 10, **characterized in that** an inlet and outlet valve (19) is located in the wall of the air cushion ring (24) and **in that** the inflatable part is inflated using an external pump or compressed air cartridge or a manual pump or compressed air cartridge integrated in the fixing means (3).

12. The device according to claim 9, **characterized in that** said highly elastic material is a foam or gel (22).

13. The device according to claim 9, **characterized in that** the two shells (23a, 23b) differ in size and **in that** the retaining clips (15) are attached to the bigger shell (23b).

14. The device according to claim 9, **characterized in that** the lock (21) can be adjusted for setting the size of the inner diameter formed by the two shells (23a, 23b).

15. The device according to claim 14, **characterized in that** the adjustable lock (21) is a locking, snap fastener, or velcro system.

16. The device according to claim 1, **characterized in that** the fixing means (3) is designed as a one-piece cylindrical, double-walled, inflatable component with a flexible inner wall and a flexible or rigid outer wall and a retaining clip (15) mounted to the outer wall, said component comprising an inlet and outlet valve (19) for inflating and deflating air.

17. The device according to claim 1, **characterized in that** the stretching rods (2) are attached to the support ring (1) using a ball joint and **in that** the retaining clips (15) are coupled to the fixing means (3).

18. The device according to claim 1, **characterized in that** the stretching rod (2) for elastic change in length consists of a threaded rod (6), an adjustment bush (7) screwed to it, and a spring-mounted spring cover (8) telescopically encompasses the adjustment bush (7), and **in that** the distal end of the threaded rod (6) comprises a stop piece (10) to prevent complete unscrewing of the adjustment bush (7).

19. The device according to claim 18, **characterized in that** markings (7a) are provided around the perimeter of the adjustment bush (7) to indicate the tensile force generated by the spring cover.

20. The device according to claim 18, **characterized in that** the stretching rod (2) can be combined of multiple extension rods (9) screwed together at various lengths.

## Revendications

1. Dispositif pour allonger de manière durable des parties corporelles oblongues, en particulier un pénis, lequel comprend une bague de soutien (1), au moins une tige d'étirement (2) fixée de manière articulée sur la bague de soutien (1), montée à effet de ressort axialement et réglable graduellement en longueur, ainsi qu'un moyen de fixation (3) retenu à l'extrémité distale de la tige d'étirement et enserrant la partie corporelle, **caractérisé en ce que** le moyen de fixation (3) est réalisé sous forme de composant (14, 18 ; 22 à 24) préformé de manière sensiblement cylindrique et entourant entièrement ou partiellement de manière souple élastique la partie corporelle concernée, avec des clips de retenue (15) susceptibles d'être enclenchés latéralement sur la périphérie de la tige d'étirement (2) après la pose du moyen de fixation (3).

2. Dispositif selon la revendication 1, **caractérisé en ce que** ledit au moins un clip de retenue (15) est réalisé sous forme de cylindre fendu de manière continue en direction longitudinale, avec des joues latérales (15a) élastiques et une plaque de butée (16) distale.

3. Dispositif selon la revendication 1, **caractérisé en ce que** les clips de retenue (15) partent de la région distale, en direction d'étirement, du moyen de fixation (3) et s'étendent au-delà de son extrémité distale.

4. Dispositif selon la revendication 1, **caractérisé en ce que** le moyen de fixation (3) est constitué par une coque de réception (14) bombée de forme concave avec des clips de retenue (15) partant latéralement depuis l'extrémité distale, et par un élément de serrage élastique (18).

5. Dispositif selon la revendication 4, **caractérisé en ce que** l'élément de serrage (18) est constitué par une partie de retenue (18a) préformée de forme bombée et élastique souple avec des bandes de serrage (18b) élastiquement extensibles partant de son extrémité, et sur les surfaces extérieures des bandes de serrage (18b) sont conformées des pattes d'enclenchement (18c) pour arrêter les bandes de serrage dans des ouvertures fendues (17) de la coque de réception (14) et des languettes de traction (18d) pour desserrer les bandes de serrage (18b) et pour limiter les forces de tension.

6. Dispositif selon la revendication 5, **caractérisé en ce que** les pattes d'enclenchement (18c) et les ouvertures fendues (17) ont des arêtes arrondies.

7. Dispositif selon la revendication 5, **caractérisé en ce que** l'épaisseur de la partie de retenue (18a) bombée est beaucoup plus grande que celle des bandes de serrage (18b) élastiques.

8. Dispositif selon la revendication 5, **caractérisé en ce que** l'élément de serrage (18) peut être fixé de façon variable en direction longitudinale sur la coque de réception (14), la longueur des ouvertures fendues (17) étant plus grande que la largeur de la bande de serrage (18b).

9. Dispositif selon la revendication 1, **caractérisé en ce que** le moyen de fixation (3) cylindrique est constitué par deux demi-coques (23a, 23b) reliées par une charnière (20) et une fermeture (21) et formant un cylindre, à la surface intérieure desquelles est appliqué un matériau hautement élastique (22, 24).

10. Dispositif selon la revendication 9, **caractérisé en ce que** le matériau hautement élastique est une bague à coussin d'air (24) gonflable et divisée dans la région de la fermeture (21).

11. Dispositif selon la revendication 10, **caractérisé en ce que** dans la paroi de la bague à coussin d'air (24) est agencée une soupape d'admission et de sortie (19) et pour gonfler la partie gonflable est prévue une pompe ou une cartouche à air comprimé externe ou une pompe ou une cartouche à air comprimée intégrée dans le moyen de fixation (3) et activable à la main.

12. Dispositif selon la revendication 9, **caractérisé en ce que** le matériau hautement élastique est une mousse expansée ou un gel (22).

13. Dispositif selon la revendication 9, **caractérisé en ce que** les deux demi-coques (23a, 23b) ont des tailles différentes, les clips de retenue étant agencés sur la demi-coque (23b) la plus grande.

14. Dispositif selon la revendication 9, **caractérisé en ce que** la fermeture (21) pour régler la taille du diamètre intérieur formé par les deux demi-coques (23a, 23b) est réalisée réglable.

15. Dispositif selon la revendication 14, **caractérisé en ce que** la fermeture (21) réglable est formée par un système de fermeture à enclenchement, à bouton-pression ou à bande autoagrippante.

16. Dispositif selon la revendication 1, **caractérisé en ce que** le moyen de fixation (3) est réalisé sous forme de composant d'un seul tenant cylindrique, avec double paroi et gonflable avec paroi intérieure flexible et paroi extérieure flexible ou rigide et avec clip de retenue (15) monté sur la paroi extérieure, lequel présente une soupape d'admission et de sortie (19) pour insuffler et laisser échapper l'air.

17. Dispositif selon la revendication 1, **caractérisé en ce que** les tiges d'étirement sont maintenues sur la bague de soutien (1) via une articulation sphérique, et les clips de retenue (15) sont montés de manière articulée sur le moyen de fixation (3).

18. Dispositif selon la revendication 1, **caractérisé en ce que** pour modifier élastiquement la longueur, la tige d'étirement (2) est constituée par une tige filetée (6), une douille de réglage (7) vissée avec celle-ci et une douille élastique (8) montée élastiquement et endurant celle-ci de façon télescopique, et sur l'extrémité distale de la tige filetée (6) est réalisée une pièce de butée (10) pour éviter le dévissage complet de la douille de réglage (7).

19. Dispositif selon la revendication 18, **caractérisé en ce que** sur la périphérie de la douille de réglage (7) sont prévus des marquages (8a) pour indiquer la force de serrage engendrée par la douille élastique (8).

20. Dispositif selon la revendication 18, **caractérisé en ce que** la tige d'étirement (2) peut être composée à partir d'une pluralité de tiges de prolongement susceptibles d'être vissées les unes aux autres à différentes longueurs.
